# EUROPEAN PATENT APPLICATION

(11) **EP 4 745 228 A1**
(43) Date of publication of application: **20.05.2026**
(21) Application number: 24213596.0
(22) Date of filing: 18.11.2024
(51) Int. Cl.: C12N 5/071

(54) **HIGH-THROUGHPUT DIFFERENTIATION METHOD OF THREE DIMENSIONAL HUMAN BLOOD VESSEL ORGANOIDS BASED ON HUMAN INDUCED PLURIPOTENT STEM CELLS**

(71) Applicant: Universitätsmedizin Greifswald, 17475 Greifswald (DE)
(72) Inventor: Skowronek, Dariush, 17491 Greifswald (DE); Felbor, Ute, 17493 Greifswald (DE); Rath, Matthias, 19055 Schwerin (DE)
(74) Representative: Altmann Stößel Dick Patentanwälte PartG mbB

(57) **Abstract**

A first aspect of the invention relates to a method for preparing a three dimensional blood vessel organoid. In a second aspect, the invention is directed to three dimensional human blood vessel organoids obtained or obtainable from the method of the first aspect of the invention. A third aspect of the invention is related to the three dimensional human blood vessel organoids for use in *(in vitro)* drug screening and/or *(in vitro)* drug testing; and/or for use in *(in vitro)* toxicity analysis; and/or for use in *(in vitro)* bioengineering; and/or for *(in vitro)* transplant preparation.

## Description

A first aspect of the invention relates to a method for preparing a three dimensional blood vessel organoid. In a second aspect, the invention is directed to three dimensional human blood vessel organoids obtained or obtainable from the method of the first aspect of the invention. A third aspect of the invention is related to the three dimensional human blood vessel organoids for use in (*in vitro*) drug sceening and/or (*in vitro*) drug testing; and/or for use in (*in vitro*) toxicity analysis; and/or for use in (*in vitro*) bioengineering; and/or for (*in vitro*) transplant preparation.

The increasing availability of advanced *in vitro* methods is raising hopes in many fields of biomedical sciences to significantly accelerate drug discovery. Organoid cultures, one of these advanced *in vitro* methods, have already enabled a wide range of new applications in basic and translational research, regenerative medicine, and drug discovery. Using specific growth factors, small molecules, extracellular matrix components, and cell culture conditions, human pluripotent stem cells can be very effectively differentiated into brain, lung, kidney, liver, and other specific organoid types. A method of generating an artificial blood vessel organoid, comprising providing stem cells capable of vascular differentiation, stimulating mesoderm differentiation in said stem cells, stimulating vascular differentiation in said stem cells, developing a cell aggregate from said stem cells, embedding said cell aggregate in a collagenous 3D matrix and stimulating vascular differentiation of the aggregate in said collagenous 3D matrix, was described by Wimmer et al. in WO 2018/229251 A1. Furthermore, the differentiation of human blood vessel organoids, which was described by Wimmer and colleagues [Wimmer RA, Leopoldi A, Aichinger M, Kerjaschki D, Penninger JM (2019) Generation of blood vessel organoids from human pluripotent stem cells. Nat Protoc 14 (11):3082-3100. doi:10.1038/s41596-019-0213-z; or Wimmer RA, Leopoldi A, Aichinger M, Wick N, Hantusch B, Novatchkova M, Taubenschmid J, Hammerle M, Esk C, Bagley JA, Lindenhofer D, Chen G, Boehm M, Agu CA, Yang F, Fu B, Zuber J, Knoblich JA, Kerjaschki D, Penninger JM (2019) Human blood vessel organoids as a model of diabetic vasculopathy. Nature 565 (7740):505-510. doi:10.1038/s41586-018-0858-8], provided new insights into the complex processes of vasculogenesis and angiogenesis in health and disease.

Despite their many advantages, vascular organoid cultures have been difficult to scale up, e.g. for high throughput studies. The methods known so far for differentiating human blood vessel organoids from human induced pluripotent stem cells were associated with the following problems and disadvantages: due to the need for manual extraction of vascular networks from the extracellular matrix, it was very labour-intensive and relied on a high level of expertise on the part of the experimenter. In addition to the high degree of delicate manual labour required, this step could also easily damage the vascular networks. Subsequently, the excised aggregates had to be cultivated together in a 6-well ultra-low attachment plate, which was not compatible with high throughput, and then transferred one by one into wells of a 96-well ultra-low attachment plate. Overall, the method was not suitable for high throughput and was by far not automatable. Furthermore, as the sowing of hiPSCs in ultra-low attachment 6-well plates was required, the hiPSCs then formed aggregates that were of variable size due to the cell culture plates used. This greatly limited the comparability between different organoids from the same synthesis run. Still further, the previous method included the use of fetal calf serum and collagen I from cattle, thus making it non-xeno free.

Cerebral cavernous malformations (CCMs), sometimes also called cavemomas, cavernous angi-omas, or cavernous hemangiomas, are leaky vascular lesions in the brain and spinal cord that can cause seizures, intracranial hemorrhages (ICH), or non-hemorrhagic focal neurological deficits (NH-FND). With a prevalence of approximately 1:200, CCM is one of the most common cerebrovascular diseases. Although most CCMs are sporadic, there is also a familial form of CCM that accounts for 6 to 7 % of all cases. Since the first description of a CCM family by H. Kufs in 1928 and the identification of the three disease genes *CCM1* (also known as *KRIT1*)*, CCM2,* and *CCM3* (also known as *PDCD10*) between 1999 and 2005, CCM research has developed rapidly. Extensive studies in different cell culture models, human CCM tissue samples as well as in mouse, zebrafish, and other model organisms have contributed significantly to our current understanding of CCM pathogenesis. It is now well established that although familial CCM is a classic autosomal dominant disorder, the inactivation of *CCM1, CCM2,* or *CCM3* is recessive at the cellular level. The deregulation of key signaling pathways that initiate CCM formation, e.g. gain of MEKK3-KLF2/4 signaling or increase of RhoA/ROCK activity, is only triggered by the biallelic inactivation of a *CCM* gene. Furthermore, next-generation sequencing of bulk and single nucleus DNA from human CCMs has shown that the proportion of cells with a biallelic *CCM* gene variant is often rather low within the vascular lesions. While *in vivo* and *in vitro* research has also led to the first phase 1 and 2 clinical trials, no pharmacological CCM therapy has yet been approved for clinical use. Thus, neurosurgical treatment often remains the only option for symptomatic CCMs, especially after a previous hemorrhage or when they cause medically refractory seizures. Since surgical resection is always associated with the risk of postoperative morbidity, the development of new CCM drugs remains a top priority in research.

Generally, a need exists to provide three dimensional blood vessel organoids by a less labor intensive method, which should also be operable with a higher throughput. Furthermore, a need exists to become as xeno-free as possible.

An object of the present invention was thus the provision of a less-work intensive method for providing three dimensional blood vessel organoids, which should also enable a high throughput and which should be as xeno-free as possible.

In a first aspect, the invention is thus directed to a method for preparing a three dimensional blood vessel organoid comprising
(a) Providing induced pluripotent stem cells (iPSCs);
(b) Providing a first cell mass culturing device CMCD(1) having at least two wells and having an arrangement of wells allowing for provision of liquid media to all wells by providing liquid medium to one single medium application site and for allowing removal of liquid media from all wells via one single medium removal site;
(c) Providing a second cell mass culturing device CMCD(2) having at least two wells, each well having an open upper section which comprises an open upper end and an open lower end, and a lower section comprising an open upper end and a bottom end, wherein the upper section and the lower section are in fluid communication with each other, and wherein the bottom end of the lower section of said at least one well is provided with a well bottom, said lower section of said at least one well having a smaller crosssectional area than the open upper section of the well;

the method comprising:
   (i) Arranging the iPSCs of (a) in the wells of CMCD(1) of (b) so that at least 100, preferably at least 200, iPSCs are present in each well, adding an aggregation medium so that the iPSCs are at least partially surrounded thereby and applying conditions suitable for aggregation of the iPSCs, thereby obtaining aggregated iPSCs;
   (ii) Removing the aggregation medium, optionally in combination with one or more washing step(s);
   (iii) Providing to the wells with aggregated iPSCs a medium suitable for inducing mesodermal differentiation in said iPSCs via the medium application site of CMCD(1), thereby obtaining aggregated iPSCs at least partially surrounded by mesoderm induction medium;
   (iv) Subjecting the aggregated iPSCs, which are at least partially surrounded by mesoderm induction medium, of (ii) to conditions suitable for inducing mesodermal differentiation (for a period of time sufficient to allow for mesoderm differentiation), thereby obtaining aggregated iPSC-derived early mesodermal cells;
   (v) Removing the mesoderm induction medium from the wells of CMCD(1) via the medium removal site and providing a medium suitable to induce vascular differentiation to the wells with aggregated iPSC-derived early mesodermal cells via the medium application site of CMCD(1), so that the aggregated iPSC-derived early mesodermal cells are at least partially surrounded by medium suitable to induce vascular differentiation;
   (vi) Subjecting the aggregated iPCS-derived early mesodermal cells, which are at least partially surrounded by medium suitable to induce vascular differentiation, to vascular differentiation conditions (for a period of time sufficient to allow for induction of vascular differentiation), thereby obtaining early vascular aggregates;
the method further comprising:
   (vii) Transferring the early vascular aggregates obtained in (vi) into wells of CMCD(2), preferably into the bottom region of the wells of CMCD(2);
   (viii) Embedding the early vascular aggregates within the wells of CMCD(2) into a matrix, said matrix comprising collagen, and solidifying the matrix having the early vascular aggregates embedded therein;
   (ix) Applying a medium suitable to induce angiogenic sprouting to the embedded early vascular aggregates obtained in (viii) and subjecting the embedded early vascular aggregates, which are at least partially surrounded by medium suitable to induce angiogenic sprouting, to sprouting-inducing conditions (for a period of time sufficient to allow for formation of vascular networks), thereby obtaining matrix embedded three dimensional vascular networks.

The method according to the present invention offers the significant advantage, especially with respect to step (x) as outline below, that it is no longer necessary to manually cut the three dimensional vascular networks off and/or to manually extract the three dimensional vascular networks. This allows for a very efficient method, which is, compared to the prior art, for the first time high throughput compatible. Thus, the inventive methods surprisingly eliminates the most labor-intensive steps and allows the differentiation of human blood vessel organoids in a 96-well format. Furthermore, as outlined in more detail below, the inventive method does not require animal collagen I and bovine calf serum, thus allowing for an at least xeno-reduced method.

Preferably, the method further comprises harvesting the three dimensional vascular networks by
(x) Removing the matrix embedded three dimensional vascular networks obtained in (ix) from the wells of CMCD(2), preferably in total, more preferably in total from all wells simultaneously, more preferably by pipetting in total from all wells simultaneously;
(xi) Transferring the matrix embedded three dimensional vascular networks into wells of a further cell mass culturing device CMCD(f);
(xii) Removing matrix from three dimensional vascular networks by dissolution with a serum free medium, preferably enhanced by mechanical movement, thereby obtaining three dimensional human blood vessel organoids free of matrix.

It is preferred that transferring of (vii) comprises
(vii. 1) optionally filling the bottom region of the wells of CMCD(2) at least partially with one or more matrix component(s) and solidifying said one or more matrix component(s) within the bottom region of the wells of CMCD(2);
(vii.2) removing at least a part of the medium suitable to induce vascular differentiation from CMCD(1) via the medium removal site;
(vii.3) removing the early vascular aggregates from the wells of CMCD(1) and putting them into wells of an intermediate cell mass culturing device CMCD(i);
(vii.4) optionally removing excess medium suitable to induce vascular differentiation from the early vascular aggregates while being in the wells of CMCD(i);
(vii.5) adding a serum free medium to the early vascular aggregates in the wells of CMCD(i), so that the early vascular aggregates in the wells of CMCD(i) are at least partially suspended in medium;
(vii.6) transferring the at least partially suspended early vascular aggregates from the wells of CMCD(i) into the wells of wells of CMCD(2) already comprising a solidified matrix in their bottom regions, thereby obtaining early vascular aggregates resting on solidified matrix.

Preferably, (viii) comprises after (vii.6)
(viii. 1) removing medium from the wells of CMCD(2) having early vascular aggregates resting on solidified matrix therein;
(viii.2) Embedding the early vascular aggregates resting on solidified matrix within the wells of CMCD(2) into matrix, said matrix comprising collagen, and solidifying the matrix having the early vascular aggregates embedded therein.

It is preferred that the first cell mass culturing device CMCD(1) comprises at least two wells, each having a culture space capable of storing the cell aggregate and culture fluid; each well having a tubular body which is disposed on the well on a plane at which the well has an opening and has an inner cavity communicating with the culture space; and the first cell mass culturing device CMCD(1) further comprising a side wall which protrudes above openings of the wells and surrounds the wells, wherein the side wall is disposed on the outside of the tubular bodies, and each tubular body having one or more communication portion(s) formed within its tubular wall, said communication portion(s) capable of discharging a culture fluid and moving the culture fluid to an area between outside of the tubular bodies but within the side wall, wherein the communication portion(s) is/are slit(s) and/or through hole(s), which prevents passage of the cell aggregate to the outside of the tubular bodies. Suitable cell mass culturing device are described, for example, in WO 2015/178413 A1. For example, the first cell mass culturing device CMCD(1) is a Prime Surface 96 Slit-well plate (Sbio, Hudson, NH, USA, #MS9096SZ).

It is preferred that the further cell mass culturing device CMCD(f) is of the same design as CMCD(1).

Preferably, for each well of the second cell mass culturing device CMCD(2), the lower section of said well has a smaller crosssectional area than the open upper section of the well, preferably a smaller cross-sectional area than the open upper end and/or the open lower end of the open upper section. The open upper section of the at least one well is configured for receiving and removing any bulk culture medium that is filled into or removed from the well. The open upper section thus constitutes the reservoir for the bulk of the culture medium within the well for the cells in culture within the well. The open upper section is therefore considered to be the liquid handling compartment of the well. The open upper end of the open upper section is configured as fluid receiving aperture of the well. In some embodiments, the lower section has a diameter smaller than the diameter of the open upper section at its open upper end, preferably a diameter in the range of from 0.1 to 5 mm, more preferably in the range of from 0.5 to 1.5 mm. In some embodiments, the well bottom of the lower section has a flat (well) bottom. Suitable cell mass culturing device are described, for example, inEP 3 317 394 B1, see especially Fig. 4. For example, the second cell mass culturing device CMCD(2) is a 96-well Akura plate (InSphero, Schlieren, Switzerland, #CS0900403),

It is preferred that the intermediate cell mass culturing device CMCD(i) is of the same design as CMCD(2).

### Aggregation

Preferably, the aggregation medium of (i) comprises a knockout DMEM/F12 medium, which preferably comprises one or more of KnockOut Serum replacement (KnockOut SR), L-glutamine (preferably Glutamax), non-essential amino acids, β-mercapthoethanol and antibiotics. The knockout DMEM/F12 is free of glutamine and free of HEPES. KnockOut SR comprises one or more compound(s) selected from the group consisting of amino acid, vitamin, transferrin or substitute, insulin or insulin substitute, trace element, collagen precursors, and albumin preloaded with lipid. KnockOut SR is preferably free of serum.

Preferably, the aggregation medium of (i) comprises a selective inhibitor of Rho-associated, coiled-coil containing protein kinase (ROCK), preferably a cyclohexane carboxamide, more preferably at least (1R,4r)-4-((R)-1-Aminoethyl)-N-(pyridin-4-yl)cyclohexanecarboxamide (Y-27632).

Conditions suitable for aggregation preferably comprise a temperature in the range of from 35 to 40°C, more preferably in the range of from 36 to 38°C, more preferably 37°C; and/or presence of at least 1%, preferably at least 2%, more preferably at least 3%, more preferably at least 4%, more preferably at least 5 %, CO₂; and/or a period of time of at least 36 hours, preferably at least 48 hours.

### Induction of mesodermal differentiation

It is preferred that the mesoderm induction medium of (iii) comprises a neurobasal medium, more preferably supplemented with N2 and/or, preferably, and, B27 (N2B27 medium). Neurobasal medium: preferably DMEM/High Glucose.

Preferably, the mesoderm induction medium of (iii) further comprises a GSK-3α/β inhibitor, preferably at least (6-[2-[[4-(2,4-dichlorophenyl)-5-(5-methyl-1H-imidazol-2-yl)pyrimidin-2-yl]amino]ethylamino]pyridine-3-carbonitrile (CHIR99021), and/or, preferably and, a bone morphogenetic protein (BMP), preferably BMP-4.

Conditions suitable for inducing mesodermal differentiation of (iv) preferably comprise a temperature in the range of from 35 to 40°C, more preferably in the range of from 36 to 38°C, more preferably 37°C; and/or presence of at least 1%, preferably at least 2%, more preferably at least 3%, more preferably at least 4%, more preferably at least 5 %, CO₂; and/or a period of time of at least 36 hours, more preferably at least 48 hours, more preferably at least 72 hours.

### Induction of vascular differentiation

It is preferred that the medium suitable for inducing vascular differentiation (v) comprises a neurobasal medium, more preferably supplemented with N2 and/or, preferably, and, B27 (N2B27 medium).

Preferably, the medium suitable for inducing vascular differentiation of (v) and (vi) further comprises VEGF-A and/or forskolin.

Vascular differentiation conditions of (vi) preferably comprise a temperature in the range of from 35 to 40°C, more preferably 36 to 38°C, more preferably 37°C; and/or presence of at least 1%, preferably at least 2%, more preferably at least 3%, more preferably at least 4%, more preferably at least 5 %, CO₂; and/or a period of time of at least 36 hours, more preferably at least 48 hours.

### Matrix

It is preferred that the matrix of (viii) comprises human collagen, more preferably human collagen I.

Preferably, the matrix of (viii) comprises Matrigel. Matrigel is solubilized basement membrane matrix secreted by Engelbreth-Holm-Swarm (EHS) mouse sarcoma cells.

Preferably, the matrix of (viii) is xeno reduced, more preferably is free of non-human collagen. Free of non-human collagen means that the matrix comprises less than 1 weight-%, preferably less than 0.1 weight-%, more preferably less than 0.01 weight-%, of non-human collagen.

The inventive method is overall preferably xeno reduced, wherein more preferably in steps (i) to (ix), more preferably in steps (i) to (x) matrigel is the only xenogen/non-human compound applied.

Preferably, the matrix of (viii) further comprises hyaluronic acid as matrix forming component, wherein preferably, the mass based ratio of hyaluronic acid to matrigel is in the range of from 0.1 : 100 to 9 : 10, preferably in the range of from 10 : 90 to 90 : 10, more preferably in the range of from 1:1 to 7:3. The hyaluronic acid is, for example, Hystem^{®} hydrogel scaffold. The hyaluronic acid is unmodified or modified, preferably thiol modified. Thiol modified hyaluronic acid is commercially available, for example, Glycosil^{®} GS222F or Glycosil^{®} GS220F (company Advanced BioMatrix, Inc.). Preferably, the hyaluronic acid, more preferably the thio modified hyaluronic acid is combined with a thiol-modified gelatin, such as Gelin-S^{®} (company Advanced BioMatrix, Inc.), an ECM protein such as laminin, collagen, or fibronectin, or with polyethylene glycol diacrylate, such as Extralink^{®}-Lite (company Advanced BioMatrix, Inc.). These components, preferably thiol modified hyaluronic acid in combination with polyethylene glycol diacrylate, are present in a buffer, such as preferably phosphate buffered saline (PBS) at a pH of about 7.4. Preferably, the weight based ratio thiol modified hyaluronic acid to polyethylene glycol diacrylate in the range of from 1:10 to 10:10, more preferably in the range of from 10:1 to 1:1, more preferably in the range of from 5:1 to 1:1. Vascular sprouting was compared between a matrix containing 100 % Matrigel (as control) and Hystem^{®} hydrogel scaffold (HS) supplemented with 35 % Matrigel. Comparison of blood vessel networks on day 10 showed that the sprouts appeared identical for the combination of HS and Matrigel vis-á-vis the control with Matrigel only.

### Angiogenic sprouting

It is preferred that the medium suitable to induce angiogenic sprouting of (ix) comprises a serum free medium and one or more of fetal bovine serum (FBS), human platelet lysate (hPL), Panexin CD (PCD), and mixtures of two or more thereof, wherein the angiogenesis medium more preferably comprises at least PCD, more preferably PCD without FBS and/or hPL. Preferably, the medium suitable to induce angiogenic sprouting of (ix) comprises at least VEGF-A and/or FGF-2. Preferably, the serum free medium comprises StemPro-34 SFM, preferably is StemPro-34 SFM.

The sprouting-inducing conditions of (viii) preferably comprise a temperature in the range of from 35 to 40°C, more preferably in the range of from 36 to 38°C, more preferably 37°C; and/or presence of at least 1%, preferably at least 2%, more preferably at least 3%, more preferably at least 4%, more preferably at least 5 %, CO₂; and/or a period of time of at least 36 hours, more preferably at least 48 hours.

Preferably, the serum-free medium used in (xii) is the same medium as the medium suitable to induce angiogenic sprouting of (ix) as defined herein above.

### Induced pluripotent stem cells (iPSCs)

It is preferred that the induced pluripotent stem cells (iPSCs) are derived or derivable from mammalian somatic cells, more preferably from mammalian somatic body tissue cells or mammalian somatic body fluid cells.

Preferably, the induced pluripotent stem cells (iPSCs) are mamalian induced pluripotent stem cells (hiPSCs), derived or derivable from mamalian blood cells, preferably from mamalian peripheral mononuclear blood cells or mamalian induced pluripotent stem cells (hiPSCs), derived or derivable from mamalian skin fibroblasts.

Preferably, the induced pluripotent stem cells (iPSCs) are human induced pluripotent stem cells (hiPSCs), derived or derivable from human blood cells, preferably from human peripheral mononuclear blood cells or human induced pluripotent stem cells (hiPSCs), derived or derivable from human skin fibroblasts.

Preferably, the induced pluripotent stem cells (iPSCs) are mouse induced pluripotent stem cells (hiPSCs), derived or derivable from mouse blood cells, preferably from mouse peripheral mononuclear blood cells or mouse induced pluripotent stem cells (hiPSCs), derived or derivable from mouse skin fibroblasts.

### In vitro

The inventive method is an *ex vivo* method or *in vitro* method, preferably an *in vitro* method.

### High throughput method

It is preferred that the inventive method is a high throughput method, wherein preferably processing is done in a multi well, preferably 96 well, plate and wherein medium exchange is done/possible automated or at least semi-automated, more preferably using a multi-pipette device, such as a 96 pipette device, for example, a VIAFLO 96 (company Integra).

### 2^{nd} aspect - Product-by-process

A second aspect of the present invention is directed to three dimensional human blood vessel organoids, obtained or obtainable from the method of the first aspect of the invention as described herein above. All details, embodiments, and preferred embodiments described in the section above related to the method of the first aspect of the invention also apply for the second aspect of the invention.

### 3^{rd} aspect - Use (medical indication)

A third aspect of the present invention is directed to three dimensional human blood vessel organoids of the second aspect of the invention as described herein above for use in research.

According to the third aspect, the invention also relates to three dimensional human blood vessel organoids of the second aspect of the invention as described herein above for use in *(in vitro)* drug screening and/or *(in vitro)* drug testing.

According to the third aspect, the invention also relates to three dimensional human blood vessel organoids of the second aspect of the invention as described herein above for use in *(in vitro)* toxicity analysis.

According to the third aspect, the invention also relates to three dimensional human blood vessel organoids of the second aspect of the invention as described herein above for use in (*in vitro*) bioengineering.

According to the third aspect, the invention also relates to three dimensional human blood vessel organoids of the second aspect of the invention as described herein above for (*in vitro*) transplant preparation.

All details, embodiments, and preferred embodiments described in the section above related to the method of the first aspect of the invention and in the section related to the second aspect of the invention also apply for the third aspect of the invention.

The invention is disclosed herein below in more details:

### RESULTS

### High-throughput-compatible and nearly xeno-free synthesis of human blood vessel organoids

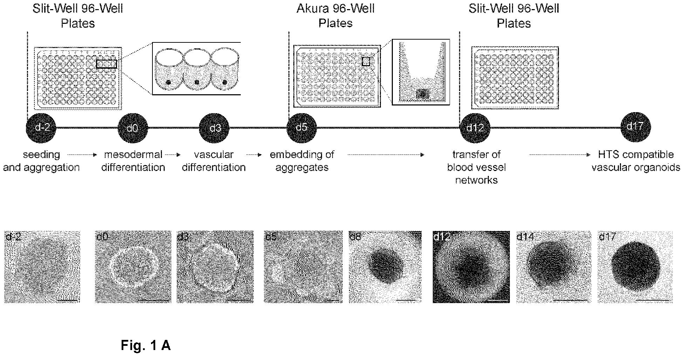

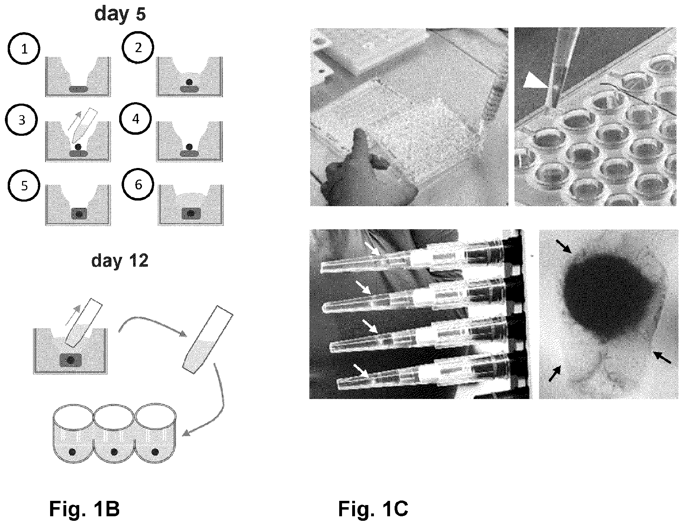

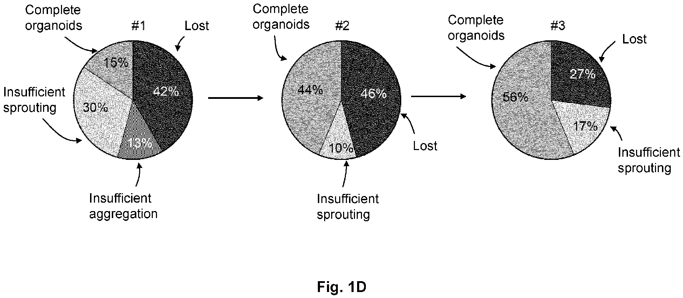

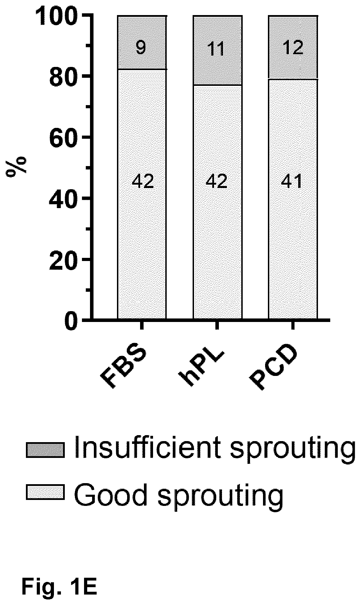

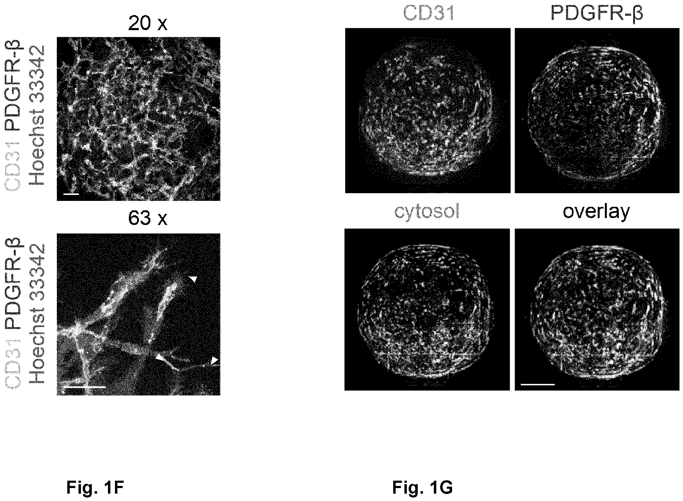

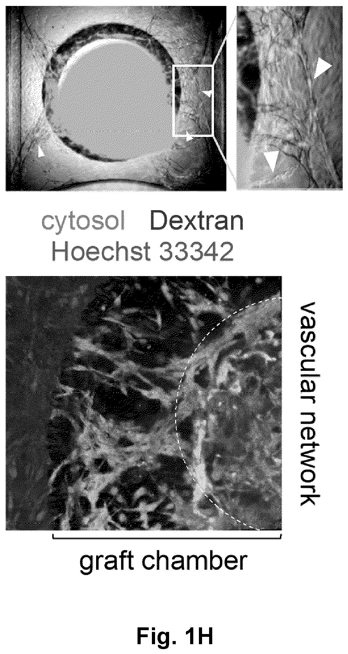

To reduce the workload of blood vessel organoid differentiation and make it more suitable for high-throughput applications, we first modified some key steps (Fig. 1A, B) of the original differentiation protocol [Wimmer RA, Leopoldi A, Aichinger M, Kerjaschki D, Penninger JM (2019) Generation of blood vessel organoids from human pluripotent stem cells. Nat Protoc 14 (11):3082-3100. doi:10.1038/s41596-019-0213-z; or Wimmer RA, Leopoldi A, Aichinger M, Wick N, Hantusch B, Novatchkova M, Taubenschmid J, Hammerle M, Esk C, Bagley JA, Lindenhofer D, Chen G, Boehm M, Agu CA, Yang F, Fu B, Zuber J, Knoblich JA, Kerjaschki D, Penninger JM (2019) Human blood vessel organoids as a model of diabetic vasculopathy. Nature 565 (7740):505-510. doi:10.1038/s41586-018-0858-8]. The use of Slit-well plates allowed precise control of the aggregate size while reducing the time spent on media exchange compared to conventional ultra-low attachment 6-well or 96-well cell culture plates. Since all wells in a Slit-well plate share one media pool, medium exchange can be performed using a 25 mL serological pipette (Fig. 1C). The new protocol also eliminates the most labor-intensive step of blood vessel organoid differentiation, namely cutting out the vascular networks from the collagen I/matrigel matrix under a sterile bench. Since the embedding of vascular aggregates on day 5 of the protocol takes place in special Akura 96 spheroid microplates, which have a conical well design with a bottom diameter of only 1 mm, the vascular networks can be directly transferred by pipetting to a new 96-well plate after sprouting. The amount of excess matrix around the vascular networks is minimal (Fig. 1B,C) and easily dissolved by regular pipetting up and down with cut pipette tips over the next seven days. The use of these special cell culture plates makes it relatively easy to learn the new protocol (Fig 1D). Besides modifying the workflow, also the reagents used in the differentiation were adapted and bovine collagen I and fetal bovine serum (FBS) were replaced with human collagen I and human platelet lysate (hPL) or Panexin CD (PCD), respectively. Since FBS, hPL, and PCD led to comparable sprouting efficiencies (Fig. 1E), it was decided to use the chemically defined serum replacement PCD for further experiments. This means that the differentiation protocol is now nearly xeno-free. Only Matrigel could not be replaced, at least not completely. Vascular networks and blood vessel organoids generated with the modified protocol are approximately 0.5 to 1.0 mm in size and display highly complex endothelial networks with associated pericytes (Fig. 1F,G). Using the OrganoPlate technology, replaced proper lumen formation in hiPSC-derived vascular networks could be demonstrated. After grafting mEGFP-tagged vascular networks onto a HUVEC bed, perfusion was visualized by introducing TMR-amino-dextran (Fig. 1H).

### Single-cell RNA sequencing (scRNA-seq) reveals different cellular compositions of WT and KO blood vessel organoids

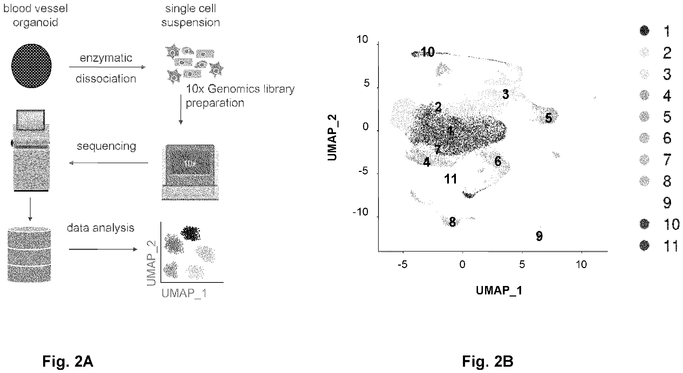

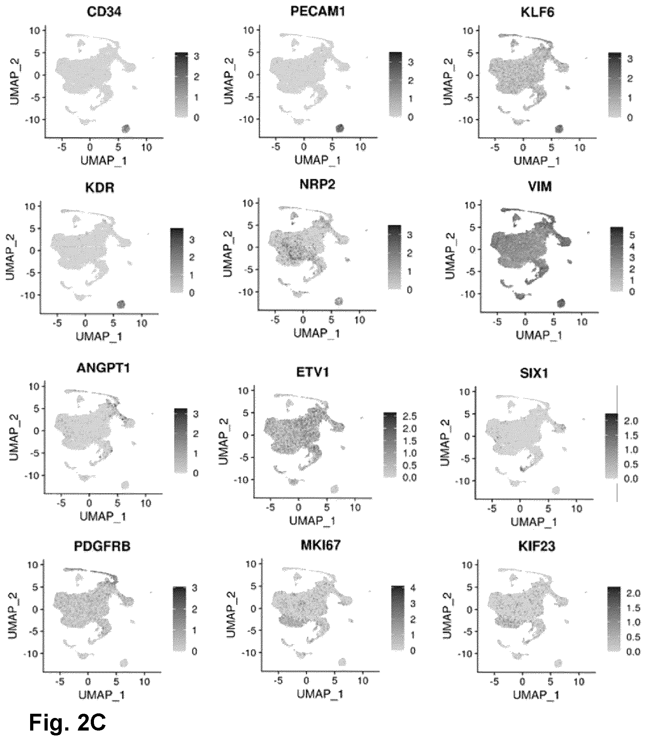

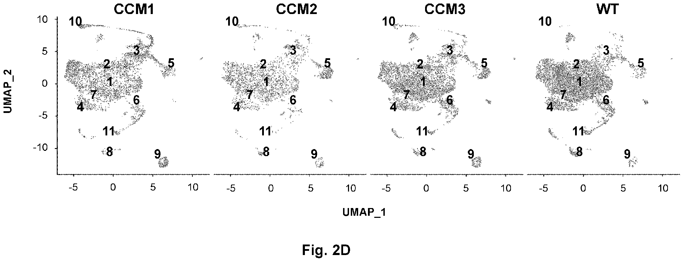

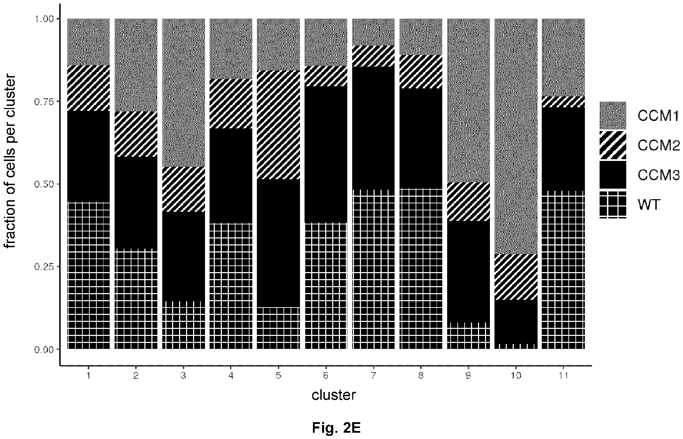

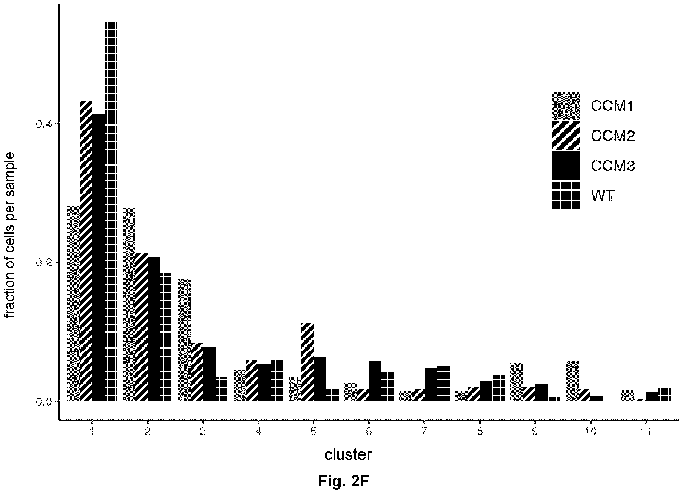

To further characterize the cellular composition and genotype-specific gene expression differences in WT and KO blood vessel organoids, scRNA-seq analysis was used (Fig. 2A). After integration of the datasets and unsupervised clustering, 11 clusters with unique gene expression signatures were identified. The visualization in a UMAP plot demonstrated good separation of the clusters (Fig. 2B). By analyzing cell-type-specificity of marker genes using the WebCSEA tool, d clusters of ECs (c1, c9), perivascular/stromal cells (c3, c5, c6, c10), cells with neuronal and glial gene expression signature (c7, c8, c11), and proliferating cells (c4) (Fig. 2B,C) were identified. Perivascular/stromal cell clusters were characterized by fibroblastic, mesenchymal, and smooth muscle cell gene expression signature. Inactivation of *CCM1, CCM2,* or *CCM3* resulted in significant shifts in the cellular composition of the organoids. While there were many overlapping effects, comparative analysis also revealed genotype-specific differences (Fig. 2D-F). For example, *CCM1* KO organoids had a marked over-representation of cells in clusters c3, c9, and c10. In contrast, *CCM2* KO organoids showed a remarkably high proportion of cells in cluster c5. Substantial changes in cellular composition were also observed in *CCM3* KO organoids. However, no specific over- or underrepresented clusters were found that distinguished *CCM3* from *CCM1* or *CCM2* KO organoids.

Overlapping and genotype-specific effects were also observed at the gene expression level. Clusters c3, c5, c6, c8, c9, and c10 showed the highest numbers of differentially expressed genes (DEGs) per genotype. Indeed, cluster c10 had the highest number of DEGs. Since this cluster also showed the largest overlap of DEGs between the three KO conditions, it was classified as the main overlap cluster. Among the most up- or downregulated genes in this fibroblast cell cluster were *ERF1, COL3A1,* and *DLK1,* which were similarly deregulated in all three KO genotypes. A GO ontology analysis of the 51 genes significantly upregulated in all three KO conditions showed a strong enrichment for genes that are involved in collagen fibril organization. A pathway analysis for the 55 DEGs downregulated in all three KO conditions revealed a more diffuse picture with weaker enrichment for different signaling pathways.

In the other clusters, the numbers of overlapping DEGs were significantly lower. We even found genotype-specific signatures in certain clusters. For example, only *CCM1* KO organoids had a high number of DEGs in cluster c8. Similar characteristic DEG clusters were also found in *CCM2* and *CCM3* KO organoids. Here, specific gene expression differences were found in clusters c3 and c9, respectively. In the EC cluster c9, a total of 193 DEGs were found in the *CCM3* KO organoids, but only 41 and 96 in *CCM1* and *CCM2* KO organoids, respectively. Only 18 DEGs were found to overlap in all three KO genotypes. These were mainly associated with angiogenesis-related processes. There was additional overlap between *CCM2* and *CCM3* KO organoids but hardly any between *CCM1* and *CCM2* or *CCM1* and *CCM3* KO organoids. The *CCM3-*specific DEGs in this cluster mainly included genes involved in cell-matrix adhesion. The *CCM1*-specific DEGs in the neuronal-like cell cluster c8 were associated with axonogenesis and neuron differentiation, and the *CCM2*-specific DEGs in the mesenchymal-like cell cluster c3 were associated with mesenchyme development and regulation of cell motility.

### Abnormal proliferation of CCM1, CCM2, and CCM3 KO cells in mosaic blood vessel organoids and endothelial co-cultures

It was previously demonstrated that *CCM3* KO cells show an abnormally high proliferation in WT/CCM3 mosaic blood vessel organoids [Rath M, Schwefel K, Malinverno M, Skowronek D, Leopoldi A, Pilz RA, Biedenweg D, Bekeschus S, Penninger JM, Dejana E, Felbor U (2022) Contact-dependent signaling triggers tumor-like proliferation of CCM3 knockout endothelial cells in co-culture with wild-type cells. Cell Mol Life Sci 79 (6):340. doi:10.1007/s00018-022-04355-6]. With our new differentiation protocol, we not only reproduced our previous result but also studied the behavior of *CCM1* KO and *CCM2* KO cells in mosaic blood vessel organoids. Wild-type hiPSCs labeled with mTagRFPT were mixed with mEGFP-labeled knockout hiPSCs (19:1 ratio) and differentiated into mosaic blood vessel organoids. In WT/*CCM3* KO and *WT*/*CCM1* KO mosaic blood vessel organoids, significantly increased mEGFP signals were observed (Fig. 8A,B). In *WT*/*CCM2* KO mosaic organoids, on the other hand, we saw significantly lower mEGFP signals. To better understand the interaction of KO and WT cells, we synthesized mosaic vascular networks and performed immunostaining analyses. A striking finding was that *CCM3* KO mosaic vascular networks appeared to be composed of much more convoluted CD31 positive endothelial and PDGFR-β positive pericyte networks than *CCM1* and *CCM2* KO mosaic networks. Another interesting finding was that VE-cadherin-positive endothelial cells appeared to be less abundant in mEGFP-labelled CCMKO cells, particularly *CCM3* KO cells.

### DISCUSSION

Although the differentiation of blood vessel organoids from hiPSCs is still a relatively new cell culture technique [ Wimmer RA, Leopoldi A, Aichinger M, Kerjaschki D, Penninger JM (2019) Generation of blood vessel organoids from human pluripotent stem cells. Nat Protoc 14 (11):3082-3100. doi:10.1038/s41596-019-0213-z], it has already had a significant impact on endothelial and vascular biology research in recent years [ Wimmer RA, Leopoldi A, Aichinger M, Wick N, Hantusch B, Novatchkova M, Taubenschmid J, Hammerle M, Esk C, Bagley JA, Lindenhofer D, Chen G, Boehm M, Agu CA, Yang F, Fu B, Zuber J, Knoblich JA, Kerjaschki D, Penninger JM (2019) Human blood vessel organoids as a model of diabetic vasculopathy. Nature 565 (7740):505-510. doi:10.1038/s41586-018-0858-8]. However, one limitation of the protocol has been its lack of scalability. In the course of the present invention, the workflow was simplified and now a high-throughput compatible protocol is present that is fully optimized for a 96-well format. Together with the use of a chemically defined, animal-free serum replacement and the possibility to perfuse the organoids. The latter two perfusion models can complement another recently described in vitro perfusion approach using a sophisticated microfluidic device, so that both simple and more complex perfusion experiments can now be realized. Since the vascularization and perfusion of organoids is also a high priority in other fields of biomedical research, these results may have an impact beyond vascular medicine.

The present invention is further illustrated by the following set of embodiments and combinations of embodiments resulting from the dependencies and back-references as indicated. In particular, it is noted that in each instance where a range of embodiments is mentioned, for example in the context of a term such as "The process of any one of embodiments 1 to 4", every embodiment in this range is meant to be explicitly disclosed for the skilled person, i.e. the wording of this term is to be understood by the skilled person as being synonymous to "The process of any one of embodiments 1, 2, 3 and 4". Further, it is explicitly noted that the following set of embodiments represents a suitably structured part of the general description directed to preferred aspects of the present invention, and, thus, suitably supports, but does not represent the claims of the present invention.
1. Method for preparing a three dimensional blood vessel organoid comprising
   (a) Providing induced pluripotent stem cells (iPSCs);
   (b) Providing a first cell mass culturing device CMCD(1) having at least two wells and having an arrangement of wells allowing for provision of liquid media to all wells by providing liquid medium to one single medium application site and for allowing removal of liquid media from all wells via one single medium removal site;
   (c) Providing a second cell mass culturing device CMCD(2) having at least two wells, each well having an open upper section which comprises an open upper end and an open lower end, and a lower section comprising an open upper end and a bottom end, wherein the upper section and the lower section are in fluid communication with each other, and wherein the bottom end of the lower section of said at least one well is provided with a well bottom, said lower section of said at least one well having a smaller crosssectional area than the open upper section of the well;

   the method comprising:
      (i) Arranging the iPSCs of (a) in the wells of CMCD(1) of (b) so that at least 100, preferably at least 200, iPSCs are present in each well, adding an aggregation medium so that the iPSCs are at least partially surrounded thereby and applying conditions suitable for aggregation of the iPSCs, thereby obtaining aggregated iPSCs;
      (ii) Removing the aggregation medium, optionally in combination with one or more washing step(s);
      (iii) Providing to the wells with aggregated iPSCs a medium suitable for inducing mesodermal differentiation in said iPSCs via the medium application site of CMCD(1), thereby obtaining aggregated iPSCs at least partially surrounded by mesoderm induction medium;
      (iv) Subjecting the aggregated iPSCs, which are at least partially surrounded by mesoderm induction medium, of (ii) to conditions suitable for inducing mesodermal differentiation (for a period of time sufficient to allow for mesoderm differentiation), thereby obtaining aggregated iPSC-derived early mesodermal cells;
      (v) Removing the mesoderm induction medium from the wells of CMCD(1) via the medium removal site and providing a medium suitable to induce vascular differentiation to the wells with aggregated iPSC-derived early mesodermal cells via the medium application site of CMCD(1), so that the aggregated iPSC-derived early mesodermal cells are at least partially surrounded by medium suitable to induce vascular differentiation;
      (vi) Subjecting the aggregated iPCS-derived early mesodermal cells, which are at least partially surrounded by medium suitable to induce vascular differentiation, to vascular differentiation conditions (for a period of time sufficient to allow for induction of vascular differentiation), thereby obtaining early vascular aggregates;
   the method further comprising:
      (vii) Transferring the early vascular aggregates obtained in (vi) into wells of CMCD(2), preferably into the bottom region of the wells of CMCD(2);
      (viii) Embedding the early vascular aggregates within the wells of CMCD(2) into a matrix, said matrix comprising collagen, and solidifying the matrix having the early vascular aggregates embedded therein;
      (ix) Applying a medium suitable to induce angiogenic sprouting to the embedded early vascular aggregates obtained in (viii) and subjecting the embedded early vascular aggregates, which are at least partially surrounded by medium suitable to induce angiogenic sprouting, to sprouting-inducing conditions (for a period of time sufficient to allow for formation of vascular networks), thereby obtaining matrix embedded three dimensional vascular networks.
2. The method of embodiment 1 further comprising harvesting the three dimensional vascular networks by
   (x) Removing the matrix embedded three dimensional vascular networks obtained in (ix) from the wells of CMCD(2), preferably in total, more preferably in total from all wells simultaneously, more preferably by pipetting in total from all wells simultaneously;
   (xi) Transferring the matrix embedded three dimensional vascular networks into wells of a further cell mass culturing device CMCD(f);
   (xii) Removing matrix from three dimensional vascular networks by dissolution with a serum free medium, preferably enhanced by mechanical movement, thereby obtaining three dimensional human blood vessel organoids free of matrix.
3. The method of embodiment 1 or 2, wherein transferring of (vii) comprises
   (vii. 1) optionally filling the bottom region of the wells of CMCD(2) at least partially with one or more matrix component(s) and solidifying said one or more matrix component(s) within the bottom region of the wells of CMCD(2);
   (vii.2) removing at least a part of the medium suitable to induce vascular differentiation from CMCD(1) via the medium removal site;
   (vii.3) removing the early vascular aggregates from the wells of CMCD(1) and putting them into wells of an intermediate cell mass culturing device CMCD(i);
   (vii.4) optionally removing excess medium suitable to induce vascular differentiation from the early vascular aggregates while being in the wells of CMCD(i);
   (vii.5) adding a serum free medium to the early vascular aggregates in the wells of CMCD(i), so that the early vascular aggregates in the wells of CMCD(i) are at least partially suspended in medium;
   (vii.6) transferring the at least partially suspended early vascular aggregates from the wells of CMCD(i) into the wells of wells of CMCD(2) already comprising a solidified matrix in their bottom regions, thereby obtaining early vascular aggregates resting on solidified matrix.
4. The method of embodiment 3, wherein (viii) comprises after (vii.6)
   (viii. 1) removing medium from the wells of CMCD(2) having early vascular aggregates resting on solidified matrix therein;
   (viii.2) Embedding the early vascular aggregates resting on solidified matrix within the wells of CMCD(2) into matrix, said matrix comprising collagen, and solidifying the matrix having the early vascular aggregates embedded therein.
5. The method of any one of embodiments 1 to 4, wherein the first cell mass culturing device CMCD(1) comprises at least two wells, each having a culture space capable of storing the cell aggregate and culture fluid; each well having a tubular body which is disposed on the well on a plane at which the well has an opening and has an inner cavity communicating with the culture space; and
   the first cell mass culturing device CMCD(1) further comprising a side wall which protrudes above openings of the wells and surrounds the wells, wherein the side wall is disposed on the outside of the tubular bodies, and each tubular body having one or more communication portion(s) formed within its tubular wall, said communication portion(s) capable of discharging a culture fluid and moving the culture fluid to an area between outside of the tubular bodies but within the side wall, wherein the communication portion(s) is/are slit(s) and/or through hole(s), which prevents passage of the cell aggregate to the outside of the tubular bodies.
6. The method of any one of embodiments 1 to 5, wherein the further cell mass culturing device CMCD(f) is of the same design as CMCD(1).
7. The method of any one of embodiments 1 to 6, wherein for each well of the second cell mass culturing device CMCD(2), the lower section of said well has a smaller crosssectional area than the open upper section of the well, preferably a smaller cross-sectional area than the open upper end and/or the open lower end of the open upper section.
8. The method of any one of embodiments 1 to 7, wherein the intermediate cell mass culturing device CMCD(i) is of the same design as CMCD(2).
9. The method of any one of embodiments 1 to 8, wherein the aggregation medium of (i) comprises a knockout DMEM/F12 medium, which preferably comprises one or more of KnockOut Serum replacement (KnockOut SR), L-glutamine (preferably Glutamax), non-essential amino acids, β-mercapthoethanol and antibiotics.
10. The method of any one of embodiments 1 to 9, wherein the aggregation medium of (i) comprises a selective inhibitor of Rho-associated, coiled-coil containing protein kinase (ROCK), preferably a cyclohexane carboxamide, more preferably at least (1R,4r)-4-((R)-1-Aminoethyl)-N-(pyridin-4-yl)cyclohexanecarboxamide (Y-27632).
11. The method of any one of embodiments 1 to 10, wherein conditions suitable for aggregation comprise a temperature in the range of from 35 to 40°C, preferably in the range of from 36 to 38°C, more preferably 37°C; and/or presence of at least 1%, preferably at least 2%, more preferably at least 3%, more preferably at least 4%, more preferably at least 5 %, CO₂; and/or a period of time of at least 36 hours, preferably at least 48 hours.
12. The method of any one of embodiments 1 to 11, wherein the mesoderm induction medium of (iii) comprises a neurobasal medium, preferably supplemented with N2 and/or, preferably, and, B27 (N2B27 medium).
13. The method of embodiment 12, wherein the wherein the mesoderm induction medium of (iii) further comprises a GSK-3α/β inhibitor, preferably at least (6-[2-[[4-(2,4-dichlorophenyl)-5-(5-methyl-1H-imidazol-2-yl)pyrimidin-2-yl]amino]ethylamino]pyridine-3-carbonitrile (CHIR99021), and/or, preferably and, a bone morphogenetic protein (BMP), preferably BMP-4.
14. The method of any one of embodiments 1 to 13, wherein conditions suitable for inducing mesodermal differentiation conditions of (iv) comprise a temperature in the range of from 35 to 40°C, preferably in the range of from 36 to 38°C, more preferably 37°C; and/or presence of at least 1%, preferably at least 2%, more preferably at least 3%, more preferably at least 4%, more preferably at least 5 %, CO₂; and/or a period of time of at least 36 hours, more preferably at least 48 hours, more preferably at least 72 hours.
15. The method of any one of embodiments 1 to 14, wherein the medium suitable for inducing vascular differentiation (v) comprises a neurobasal medium, preferably supplemented with N2 and/or, preferably, and, B27 (N2B27 medium).
16. The method of embodiment 15, wherein the medium suitable for inducing vascular differentiation of (v) and (vi) further comprises VEGF-A and/or forskolin.
17. The method of any one of embodiments 1 to 16, wherein vascular differentiation conditions of (vi) comprise a temperature in the range of from 35 to 40°C, preferably 36 to 38°C, more preferably 37°C; and/or presence of at least 1%, preferably at least 2%, more preferably at least 3%, more preferably at least 4%, more preferably at least 5 %, CO₂; and/or a period of time of at least 36 hours, more preferably at least 48 hours.
18. The method of any one of embodiments 1 to 17, wherein the matrix of (viii) comprises human collagen, preferably human collagen I.
19. The method of any one of embodiments 1 to 18, wherein the matrix of (viii) comprises Matrigel.
20. The method of any one of embodiments 1 to 19, wherein the matrix of (viii) is xeno reduced, preferably is free of non-human collagen.
21. The method of any one of embodiments 1 to 20 being xeno reduced, wherein preferably in steps (i) to (ix), more preferably in steps (i) to (x) matrigel is the only xenogen/non-human compound applied.
22. The method of any one of embodiments 1 to 21, wherein the matrix of (viii) further comprises hyaluronic acid as matrix forming component, wherein preferably, the mass based ratio of hyaluronic acid to matrigel is in the range of from 0.1 : 100 to 9 : 10, preferably in the range of from 10 : 90 to 90 : 10, more preferably in the range of from 1:1 to 7:3
23. The method of any one of embodiments 1 to 22, wherein the medium suitable to induce angiogenic sprouting of (ix) comprises a serum free medium and one or more of fetal bovine serum (FBS), human platelet lysate (hPL), Panexin CD (PCD), and mixtures of two or more thereof, wherein the angiogenesis medium preferably comprises at least PCD, more preferably PCD without FBS and/or hPL.
24. The method of embodiment 23, wherein the medium suitable to induce angiogenic sprouting of (ix) comprises at least VEGF-A and/or FGF-2.
25. The method of embodiment 24, wherein the serum free medium comprises StemPro-34 SFM, preferably is StemPro-34 SFM.
26. The method of any one of embodiments 1 to 25, wherein the sprouting-inducing conditions of (viii) comprise a temperature in the range of from 35 to 40°C, preferably in the range of from 36 to 38°C, more preferably 37°C; and/or presence of at least 1%, preferably at least 2%, more preferably at least 3%, more preferably at least 4%, more preferably at least 5 %, CO₂; and/or a period of time of at least 36 hours, more preferably at least 48 hours.
27. The method of any one of embodiments 1 to 25, wherein the serum-free medium used in (xii) is the same medium as the medium suitable to induce angiogenic sprouting of (ix) as defined in any one of embodiments 23 to 26.
28. The method of any one of embodiments 1 to 27, wherein the induced pluripotent stem cells (iPSCs) are derived or derivable from mammalian somatic cells, preferably from mammalian somatic body tissue cells or mammalian somatic body fluid cells.
29. The method of any one of embodiments 1 to 28, wherein the induced pluripotent stem cells (iPSCs) are mamalian induced pluripotent stem cells (hiPSCs), derived or derivable from mamalian blood cells, preferably from mamalian peripheral mononuclear blood cells or mamalian induced pluripotent stem cells (hiPSCs), derived or derivable from mamalian skin fibroblasts.
30. The method of any one of embodiments 1 to 29, wherein the induced pluripotent stem cells (iPSCs) are human induced pluripotent stem cells (hiPSCs), derived or derivable from human blood cells, preferably from human peripheral mononuclear blood cells or human induced pluripotent stem cells (hiPSCs), derived or derivable from human skin fibroblasts.
31. The method of any one of embodiments 1 to 29, wherein the induced pluripotent stem cells (iPSCs) are mouse induced pluripotent stem cells (hiPSCs), derived or derivable from mouse blood cells, preferably from mouse peripheral mononuclear blood cells or mouse induced pluripotent stem cells (hiPSCs), derived or derivable from mouse skin fibroblasts.
32. The method of any one of embodiments 1 to 31, which is an *ex vivo* method or *in vitro* method, preferably an *in vitro* method.
33. The method of any one of embodiments 1 to 32, being a high throughput method, wherein preferably processing is done in a multi well, preferably 96 well, plate and wherein medium exchange is done automated or at least semi-automated, more preferably using a multi-pipette device.
34. Three dimensional human blood vessel organoids obtained or obtainable from the method of any one of embodiments 1 to 33.
35. Three dimensional human blood vessel organoids of embodiment 34 for use in research.
36. Three dimensional human blood vessel organoids of embodiment 34 for use in *(in vitro)* drug sceening and/or *(in vitro)* drug testing.
37. Three dimensional human blood vessel organoids of embodiment 34 for use in *(in vitro)* toxicity analysis.
38. Three dimensional human blood vessel organoids of embodiment 34 for use in *(in vitro)* bioengineering.
39. Three dimensional human blood vessel organoids of embodiment 34 for *(in vitro)* transplant preparation.

### MATERIALS AND METHODS

### Cell culture

Human induced pluripotent stem cells (hiPSCs) were cultured in 6-well plates (Greiner Bio-One, Frickenhausen, Germany, #657160) using Essential 8 Flex medium (Thermo Fisher Scientific, Waltham, MA, USA, #A2858501). Cell culture dishes were pre-coated with growth factor-reduced Matrigel (Corning Inc., Corning, NY, USA, #356231). HiPSCs were thawed and plated at a density of 10,000 cells/cm² in Matrigel-coated 6-well plates in the presence of RevitaCell supplement (1:100) (Thermo Fisher Scientific, #A2644501). HiPSC cultures were regularly tested negative for mycoplasma contamination by PCR. The hiPSC lines AICS-0036-006 [WTC-mEGFP-Safe harbor locus (AAVS1)-cl6 (mono-allelic tag); hPSCreg ID: UCSFi001-A-12; RRID:CVCL_JM19], AICS-0054-091 [WTC-mTagRFPT-CAAX-Safe harbor locus (AAVS1)-cl91 (mono-allelic tag); hPSCreg ID: UCSFi001-A-23; RRID:CVCL_VK84] and AICS-0016-184 [WTC-mEGFP-ACTB-cl184 (mono-allelic tag); hPSCreg ID: UCSFi001-A-3; RRID:CVCL_JM16], which are part of the Allen Cell Collection (Allen Institute for Cell Science, Seattle, WA, USA), were purchased from the Coriell Institute (Camden, NJ, USA). *CCM1* and *CCM2* knockout (KO) AICS-0036-006 hiPSCs as well as *CCM1, CCM2,* and *CCM3* KO AICS-0016-184 hiPSCs were generated in this study following our established protocols [[Rath M, Schwefel K, Malinverno M, Skowronek D, Leopoldi A, Pilz RA, Biedenweg D, Bekeschus S, Penninger JM, Dejana E, Felbor U (2022) Contact-dependent signaling triggers tumor-like proliferation of CCM3 knockout endothelial cells in co-culture with wild-type cells. Cell Mol Life Sci 79 (6):340. doi:10.1007/s00018-022-04355-6; Pilz RA, Skowronek D, Hamed M, Weise A, Mangold E, Radbruch A, Pietsch T, Felbor U, Rath M (2022) Using CRISPR/Cas9 genome editing in human iPSCs for deciphering the pathogenicity of a novel CCM1 transcription start site deletion. Front Mol Biosci 9:953048. doi:10.3389/fmolb.2022.953048]. The generation of *CCM3* KO AICS-0036-006 hiPSCs has been described earlier [Rath M, Schwefel K, Malinverno M, Skowronek D, Leopoldi A, Pilz RA, Biedenweg D, Bekeschus S, Penninger JM, Dejana E, Felbor U (2022) Contact-dependent signaling triggers tumor-like proliferation of CCM3 knockout endothelial cells in co-culture with wild-type cells. Cell Mol Life Sci 79 (6):340. doi:10.1007/s00018-022-04355-6]. *"CCM1* KO", *"CCM2* KO", and *"CCM3* KO" hereafter refer to KO cells derived from AICS-0036-006-hiPSCs unless otherwise specified. For CRISPR/Cas9 genome editing, single guide RNA (sgRNA):Cas9 ribonucleoprotein complexes with the following target sequences were used: *CCM1* 5'-GGAGCTCCTAGACCAAAGTA-3'; *CCM2* 5'-GGTCAGTTAACGTCCATACC-3'; *CCM3* 5'-CAACTCACCTCATTAAACAC-3' (Integrated DNA Technologies, Coralville, IA, USA). The introduction of homozygous or compound heterozygous loss-of-function variants by CRISPR/Cas9-editing in the generated clones was verified by amplicon-based next-generation sequencing. HiPSC quality control was performed by karyo-typing and immunofluorescence analysis of the pluripotency markers OCT4, SSEA4, SOX2, and TRA-1-60 using the Pluripotent Stem Cell 4-Marker Immunocytochemistry Kit (Thermo Fisher Scientific, #A24881) as previously described [Pilz RA, Skowronek D, Hamed M, Weise A, Mangold E, Radbruch A, Pietsch T, Felbor U, Rath M (2022) Using CRISPR/Cas9 genome editing in human iPSCs for deciphering the pathogenicity of a novel CCM1 transcription start site deletion. Front Mol Biosci 9:953048. doi:10.3389/fmolb.2022.953048].

### Generation of hiPSC, mesodermal, and vascular aggregates

For the generation of hiPSC aggregates and their differentiation into vascular aggregates, the previously described protocol of Wimmer and colleagues [Wimmer RA, Leopoldi A, Aichinger M, Kerjaschki D, Penninger JM (2019) Generation of blood vessel organoids from human pluripotent stem cells. Nat Protoc 14 (11):3082-3100. doi:10.1038/s41596-019-0213-z; or Wimmer RA, Leopoldi A, Aichinger M, Wick N, Hantusch B, Novatchkova M, Taubenschmid J, Hammerle M, Esk C, Bagley JA, Lindenhofer D, Chen G, Boehm M, Agu CA, Yang F, Fu B, Zuber J, Knoblich JA, Kerjaschki D, Penninger JM (2019) Human blood vessel organoids as a model of diabetic vasculopathy. Nature 565 (7740):505-510. doi:10.1038/s41586-018-0858-8] was modified to allow aggregate formation in a 96-well format. Briefly, on day -2 hiPSCs were detached and dissociated using StemPro Accutase (Thermo Fisher Scientific, #A1110501). Cells were counted and diluted to a concentration of 1,000 cells/well in fresh aggregation medium [ Wimmer RA, Leopoldi A, Aichinger M, Kerjaschki D, Penninger JM (2019) Generation of blood vessel organoids from human pluripotent stem cells. Nat Protoc 14 (11):3082-3100. doi:10.1038/s41596-019-0213-z; or Wimmer RA, Leopoldi A, Aichinger M, Wick N, Hantusch B, Novatchkova M, Taubenschmid J, Hammerle M, Esk C, Bagley JA, Lindenhofer D, Chen G, Boehm M, Agu CA, Yang F, Fu B, Zuber J, Knoblich JA, Kerjaschki D, Penninger JM (2019) Human blood vessel organoids as a model of diabetic vasculopathy. Nature 565 (7740):505-510. doi:10.1038/s41586-018-0858-8] containing 50 µM Y-27632 (Stemcell Technologies, Vancouver, Canada, #72304). Cells were seeded in 100 µL medium per well in ultra-low attachment Prime Surface 96 Slit-well plates (Sbio, Hudson, NH, USA, #MS9096SZ). Plates were centrifuged at 300 × g for 3 min at room temperature and incubated at 37 °C and 5 % CO2 for 48 hours to promote effective hiPSC aggregation. On day 0, the PrimeSurface 96 Slit-well plates were washed by adding 25 mL of N2B27 medium to the plate using a 25 mL serological pipette. The plate was gently tilted to all sides to distribute the medium evenly. The medium was removed by titling the 96 Slit-well plate and slowly aspirating it from one corner of the plate with a 25 mL serological pipette. Next, 25 mL of N2B27 medium + 12 µM CHIR99021 (Tocris Bioscience, Bristol, United Kingdom, #4423) + 30 ng/mL BMP-4 (Miltenyi Biotec, Bergisch Glad-bach, Germany, #130-111-164) were added. The cells were incubated at 37 °C and 5 % CO2 for 72 hours to induce mesodermal differentiation. On day 3, 25 mL of the old medium were aspirated, the aggregates were washed as described above and 25 mL of N2B27 medium + 100 ng/mL VEGF-A (Peprotech, Hamburg, Germany, #100-20-50) + 2 µM forskolin (Miltenyi Biotec, #130-117-341) were added. The cells were incubated at 37 °C and 5 % CO2 for 48 hours to induce vascular differentiation. To study aggregation efficiency KO and WT hiPSCs were seeded with different cell densities ranging from 200 cells/well to 1800 cells/well. Following the protocol of mesodermal and endothelial differentiation described above, the aggregates were fixed with 4 % PFA and imaged on days 0, 3, and 5 (= hiPSC, mesodermal, and endothelial aggregates, respectively). The imaging was performed using an EVOS M5000 imaging systems microscope (Thermo Fisher Scientific). The size of the aggregates was determined using FIJI v.1.54 [Schindelin J, Arganda-Carreras I, Frise E, Kaynig V, Longair M, Pietzsch T, Preibisch S, Rueden C, Saalfeld S, Schmid B, Tinevez JY, White DJ, Hartenstein V, Eliceiri K, Tomancak P, Cardona A (2012) Fiji: an open-source platform for biological-image analysis. Nat Methods 9 (7):676-682. doi:10.1038/nmeth.2019]. The following primary and secondary antibodies were used for immunofluorescent evaluation: ZO-1 (Novus Biologicals, Centennial, CO, USA, 1:100, #NBP1-85047), anti-rabbit IgG Alexa 647 (Thermo Fisher Scientific, 1:200 #A21246).

### Generation of human blood vessel organoids

For the generation of vascular networks and blood vessel organoids, the previously described protocol by Wimmer and colleagues [Wimmer RA, Leopoldi A, Aichinger M, Kerjaschki D, Penninger JM (2019) Generation of blood vessel organoids from human pluripotent stem cells. Nat Protoc 14 (11):3082-3100. doi:10.1038/s41596-019-0213-z; Wimmer RA, Leopoldi A, Aichinger M, Wick N, Hantusch B, Novatchkova M, Taubenschmid J, Hammerle M, Esk C, Bagley JA, Lindenhofer D, Chen G, Boehm M, Agu CA, Yang F, Fu B, Zuber J, Knoblich JA, Kerjaschki D, Penninger JM (2019) Human blood vessel organoids as a model of diabetic vasculopathy. Nature 565 (7740):505-510. doi:10.1038/s41586-018-0858-8] was modified as follows: On day 5, vascular aggregates were transferred to a 96-well Akura plate (InSphero, Schlieren, Switzerland, #CS0900403) and embedded in a human collagen I/Matrigel matrix. The following amounts of human collagen I (Advanced BioMatrix, Carlsbad, CA, USA, #5007-20ML) and Matrigel solution were used for one 96-well Akura plate: 490 µL each for the first and second matrix layers (367,5 µL of human collagen I solution + 122,5 µL of Matrigel). Matrigel and human collagen I were kept on ice. To form the first matrix layer, 3 µL of human collagen I/Matrigel solution was added to each well of an empty Akura 96-well plate. The plate was incubated at 37 °C for 2 h to solidify the matrix while a second Akura plate was prepared as a transfer plate. To avoid air bubbles at the bottom of the transfer plate, the wells were washed with StemPro-34 serum-free medium (StemPro-34 SFM, Thermo Fisher Scientific, #10639011). After removing 10 mL of the N2B27 medium from the PrimeSurface 96 Slit-well plate containing the vascular aggregates, a multichannel pipette with cut pipette tips was used to aspirate the vascular aggregates in 70 µL medium from the bottom of the Slit-Well plate. They were transferred to the transfer plate and excess medium was removed. Afterwards, 50 µL of StemPro-34 SFM was added to each well before 40 µL of medium containing the aggregates was transferred into the Akura plate containing the first collagen I/Matrigel matrix layer using a multichannel pipette with cut pipette tips. Once again, as much medium as possible was aspirated before 5 µL of human collagen I/Matrigel solution was slowly added as a second matrix layer embedding the aggregates. For solidification of the matrix, the plate was incubated for 2 hours at 37 °C. Meanwhile, StemPro-34 SFM containing 15 % Panexin CD (PAN Biotech, Aidenbach, Germany, #P04-930500) was prepared in a Falcon tube and heated to 37 °C in a water bath for 30 minutes. 80 µL of StemPro-34 SFM supplemented with 15 % Panexin CD, 100 ng/mL FGF-2 (Miltenyi Biotec, #130-093-564), and 100 ng/mL VEGF-A were carefully added to the center of each well. The plate was incubated at 37 °C and 5 % CO₂ to induce sprouting. The medium was replaced on days 7 and 10. On day 12, the matrix plugs with the vascular networks were transferred without manual extraction from the gel to individual wells of a PrimeSurface 96 Slit-well plate by pipetting. Medium was changed as previously described by adding 25 mL of StemPro-34 SFM supplemented with 15 % Panexin CD, 100 ng/mL FGF-2 and 100 ng/mL VEGF-A to the Slit-well plate. Over the next two days, the networks were regularly pipetted up and down with cut pipette tips to promote the dissolution of excess matrix. The medium was exchanged again on day 14. On day 17, the blood vessel organoids were ready for following analyses. To generate mosaic blood vessel organoids, mTagRFPT-tagged AICS-0054 WT hiPSCs and *CCM1* KO, *CCM2* KO or *CCM3* KO mEGFP-tagged AICS-0036 hiPSCS were mixed in a 19:1 ratio and differentiated to blood vessel organoids as described above. For immunofluorescence analysis, vascular networks and blood vessel organoids were fixed in 1 % PFA for 30 min or 1 h, respectively. Antibody staining was performed as described elsewhere [Wimmer RA, Leopoldi A, Aichinger M, Kerjaschki D, Penninger JM (2019) Generation of blood vessel organoids from human pluripotent stem cells. Nat Protoc 14 (11):3082-3100. doi:10.1038/s41596-019-0213-z;]. The following primary and secondary antibodies were used: CD31 (RnD Systems, Minneapolis, MN, USA, #BBA7, 1:50), PDGFR-β (Cell Signaling Technology, Danvers, MA, USA, #3169S, 1:50), VE-cadherin (Cell Signaling Technology, #2500S, 1:400), and collagen IV (Novus Biologicals, #NB120-6586, 1:100), anti-Mouse IgG Fluor 350 (Thermo Fisher Scientific, #A-11045), anti-Rabbit IgG Alexa Fluor 647 (Thermo Fisher Scientific, 1:200, #A21246), anti-Mouse IgG Alexa 555 (Abcam, Cambridge, England, 1:200, #ab150114). Nuclei were stained with Hoechst 33342.

Imaging was performed using an Operetta CLS imaging system (PerkinElmer, Waltham, MA, USA) in non-confocal mode or a Stellaris 8 (Leica, Wetzlar, Germany) microscope.

### Perfusion of human blood vessel organoids in OrganoPlates Graft plates

*In vitro* perfusion assays were performed with OrganoPlate Graft plates (Mimetas, Oegstgeest, Netherlands, #6401-400-B). Gel preparation was performed according to the manufacturer's instructions. 3 µL of gel containing 4 mg/mL rat tail Collagen I (Ibidi, Gräfelfing, Germany, #50201), 100 mM HEPES (Thermo Fisher Scientific, #15630106), and 3.7 mg/mL NaHCO₃ (Merck, Darmstadt, Germany, #6329) were added to the gel inlet of the OrganoPlate. For polymerization of the matrix, the plate was incubated for 15 minutes at 37 °C and 5 % CO₂. Human umbilical vein endothelial cells (HUVECs, PromoCell, Heidelberg, Germany) were cultured in endothelial cell growth medium (ECGM, PromoCell) supplemented with 10 % fetal bovine serum (FBS) (Thermo Fisher Scientific, #A5670701). Following the manufacturer's instructions, 2 µL of 1 × 10⁴ cells/µL were seeded into the perfusion inlets of the Organoplate. After adding 50 µL of medium to the perfusion inlets, the plate was incubated for 1 hour at 37 °C and 5 % CO₂. After cell attachment, 50 µL of medium was added to the perfusion outlets, and the plate was placed on an interval MIMETAS rocker with an inclination of 14° and an interval of 8 minutes. Medium was replaced every two to three days. Cells were cultured for three days until tube formation was completed. To initiate sprouting, 50 µL of medium mixed with an angiogenic cocktail of 37.5 ng/mL VEGF-A, 37.5 ng/mL FGF-2, 37.5 ng/mL rhMCP-1 (ImmunoTools, Friesoythe, Germany, #11343384), 37.5 ng/mL rhHGF (ImmunoTools, #11343413), 250 nM S1P (Sigma Aldrich, St. Louis, MO, USA, #S9666) and 37.5 ng/mL PMA (Sigma Aldrich, # P1585) were added to the graft chamber according to the manufacturer's instruction. To graft hiPSC-derived vascular networks in the OrganoPlates, medium was removed from all channels after six days of sprouting. Next, perfusion inlets and outlets were filled with ECGM supplemented with 10 % FBS. StemPro-34 SFM containing 15 % PCD and the previously described angiogenic cocktail was added to the graft chamber. Finally, the vascular networks were transferred into the graft chamber using cut pipette tips. Medium was replaced every two to three days. After five days the perfusion of the vascular network was visualized by adding medium containing 0.5 mg/mL 150 kDA tetramethylrhodamine (TMR)-amino dextran (Fina Biosolutions LLC, Rockville, MD, USA, #TMR-Amdex150K) to the left perfusion inlet and outlet. After 15 minutes the plate was fixed using 4 % PFA for 30 minutes and imaged according to the manufacturer's instructions.

### Single cell RNA sequencing (scRNA-Seq)

After blood vessel organoid synthesis, ten organoids per genotype were pooled and enzymatic dissociation was performed as follows: Blood vessel organoids were transferred to a sterile Petri dish and minced with a scalpel. The minced organoids were then suspended in 1 mL enzymatic mix containing 4 mg Liberase TH solution (Sigma Aldrich), 30 mg Dispase II (Sigma Aldrich), and 0.5 mg DNase I solution (New England Biolabs, Ipswich MA, USA) in PBS and incubated at 37 °C and 5 % CO₂ for 30 minutes. The dissociated blood vessel organoids were mixed and passed through a 70 µm cell strainer. 3 mL of DMEM/F-12 (Thermo Fisher Scientific, #11330032) with 10 % FBS (Thermo Fisher Scientific, #A5670701) was added to stop the enzymatic reaction. Cells were centrifuged at 300 × g for 5 minutes, resuspended in StemPro-34 medium with 10 % DMSO, and frozen in liquid nitrogen for shipment. Library preparation for scRNA-Seq was performed with the Chromium Single Cell Next GEM 3' Reagent Kit v3.1 (10x Genomics, Pleasanton, CA, USA) on a Single Cell 3' device following manufacturer's instructions. Sequencing was performed on a Novaseq 6000 sequencer (Illumina, San Diego, CA, USA). Cellranger version 7.0.1 was used to generate read data, demultiplex single cells, perform sequence alignment, and generate raw count data. Further filtering of raw data, generation of clusters, and analysis of gene expression differences were performed with Seurat 4.0.4. In brief, cells with a UMI count under 2500 and less than 600 detected genes were removed and the mitochondrial DNA and ribosomal RNA cut off values were set to 10 % and 5 %, respectively. Seurat SCTransform was further used to normalize samples. Data was integrated using a mutual nearest neighbors (MNN) approach and unsupervised cell clustering was performed with the Leiden algorithm. Web-based Cell-type-Specific Enrichment Analysis of Genes (WebCSEA) was applied to identify enriched cell types of generated clusters [Dai Y, Hu R, Liu A, Cho KS, Manuel AM, Li X, Dong X, Jia P, Zhao Z (2022) WebCSEA: web-based cell-type-specific enrichment analysis of genes. Nucleic Acids Res 50 (W1):W782-W790. doi:10.1093/nar/gkac392] using the protein-coding marker genes with a log₂FC ≥ 0.5 for each cluster. For each cluster, The CZ CELLxGENE Discover browser [Abdulla S, Aevermann B, Assis P, Badajoz S, Bell SM, Bezzi E, Cakir B, Chaffer J, Chambers S, Michael Cherry J, Chi T, Chien J, Dorman L, Garcia-Nieto P, Gloria N, Hastie M, Hegeman D, Hilton J, Huang T, Infeld A, Istrate A-M, Jelic I, Katsuya K, Kim YJ, Liang K, Lin M, Lombardo M, Marshall B, Martin B, McDade F, Megill C, Patel N, Predeus A, Raymor B, Robatmili B, Rogers D, Rutherford E, Sadgat D, Shin A, Small C, Smith T, Sridharan P, Tarashansky A, Tavares N, Thomas H, Tolopko A, Urisko M, Yan J, Yeretssian G, Zamanian J, Mani A, Cool J, Carr A (2023) CZ CELL×GENE Discover: A single-cell data platform for scalable exploration, analysis and modeling of aggregated data. bio-Rxiv:2023.2010.2030.563174. doi:10.1101/2023.10.30.563174] was used to visualize the tissue and cell type-specific expression levels of the top 10 marker genes in specific clusters. Differentially expressed genes (DEG) of individual clusters were analyzed with the ShinyGO tool [Ge SX, Jung D, Yao R (2020) ShinyGO: a graphical gene-set enrichment tool for animals and plants. Bioinformatics 36 (8):2628-2629. doi:10.1093/bioinformatics/btz931] and subjected to gene set enrichment analyses with the GO cellular process and molecular function gene sets.

### Statistical analysis

Statistical analysis was performed in GraphPad Prism 8 (GraphPad Software, USA). Normality was assessed using the Shapiro-Wilk test. For normally distributed data, statistical significance was evaluated using two-way ANOVA with Šídák's multiple comparison post hoc test or parametric t-test. For non-normally distributed data, the Mann-Whitney test was used, as explained in the figure legends. ns = P ≥ 0.05; * = P < 0.05; ** = P < 0.01; *** = P < 0.001.

## Claims

1. Method for preparing a three dimensional blood vessel organoid comprising
(a) Providing induced pluripotent stem cells (iPSCs);
(b) Providing a first cell mass culturing device CMCD(1) having at least two wells and having an arrangement of wells allowing for provision of liquid media to all wells by providing liquid medium to one single medium application site and for allowing removal of liquid media from all wells via one single medium removal site;
(c) Providing a second cell mass culturing device CMCD(2) having at least two wells, each well having an open upper section which comprises an open upper end and an open lower end, and a lower section comprising an open upper end and a bottom end, wherein the upper section and the lower section are in fluid communication with each other, and wherein the bottom end of the lower section of said at least one well is provided with a well bottom, said lower section of said at least one well having a smaller crosssectional area than the open upper section of the well;
the method comprising:
(i) Arranging the iPSCs of (a) in the wells of CMCD(1) of (b) so that at least 100, preferably at least 200, iPSCs are present in each well, adding an aggregation medium so that the iPSCs are at least partially surrounded thereby and applying conditions suitable for aggregation of the iPSCs, thereby obtaining aggregated iPSCs;
(ii) Removing the aggregation medium, optionally in combination with one or more washing step(s);
(iii) Providing to the wells with aggregated iPSCs a medium suitable for inducing mesodermal differentiation in said iPSCs via the medium application site of CMCD(1), thereby obtaining aggregated iPSCs at least partially surrounded by mesoderm induction medium;
(iv) Subjecting the aggregated iPSCs, which are at least partially surrounded by mesoderm induction medium, of (ii) to conditions suitable for inducing mesodermal differentiation, thereby obtaining aggregated iPSC-derived early mesodermal cells;
(v) Removing the mesoderm induction medium from the wells of CMCD(1) via the medium removal site and providing a medium suitable to induce vascular differentiation to the wells with aggregated iPSC-derived early mesodermal cells via the medium application site of CMCD(1), so that the aggregated iPSC-derived early mesodermal cells are at least partially surrounded by medium suitable to induce vascular differentiation;
(vi) Subjecting the aggregated iPCS-derived early mesodermal cells, which are at least partially surrounded by medium suitable to induce vascular differentiation, to vascular differentiation conditions, thereby obtaining early vascular aggregates;
the method further comprising:
(vii) Transferring the early vascular aggregates obtained in (vi) into wells of CMCD(2), preferably into the bottom region of the wells of CMCD(2);
(viii) Embedding the early vascular aggregates within the wells of CMCD(2) into a matrix, said matrix comprising collagen, and solidifying the matrix having the early vascular aggregates embedded therein;
(ix) Applying a medium suitable to induce angiogenic sprouting to the embedded early vascular aggregates obtained in (viii) and subjecting the embedded early vascular aggregates, which are at least partially surrounded by medium suitable to induce angiogenic sprouting, to sprouting-inducing conditions, thereby obtaining matrix embedded three dimensional vascular networks.

2. The method of claim 1 further comprising harvesting the three dimensional vascular networks by
(x) Removing the matrix embedded three dimensional vascular networks obtained in (ix) from the wells of CMCD(2), preferably in total, more preferably in total from all wells simultaneously, more preferably by pipetting in total from all wells simultaneously;
(xi) Transferring the matrix embedded three dimensional vascular networks into wells of a further cell mass culturing device CMCD(f);
(xii) Removing matrix from three dimensional vascular networks by dissolution with a serum free medium, preferably enhanced by mechanical movement, thereby obtaining three dimensional human blood vessel organoids free of matrix.

3. The method of claim 1 or 2, wherein transferring of (vii) comprises
(vii. 1) optionally filling the bottom region of the wells of CMCD(2) at least partially with one or more matrix component(s) and solidifying said one or more matrix component(s) within the bottom region of the wells of CMCD(2);
(vii.2) removing at least a part of the medium suitable to induce vascular differentiation from CMCD(1) via the medium removal site;
(vii.3) removing the early vascular aggregates from the wells of CMCD(1) and putting them into wells of an intermediate cell mass culturing device CMCD(i);
(vii.4) optionally removing excess medium suitable to induce vascular differentiation from the early vascular aggregates while being in the wells of CMCD(i);
(vii.5) adding a serum free medium to the early vascular aggregates in the wells of CMCD(i), so that the early vascular aggregates in the wells of CMCD(i) are at least partially suspended in medium;
(vii.6) transferring the at least partially suspended early vascular aggregates from the wells of CMCD(i) into the wells of wells of CMCD(2) already comprising a solidified matrix in their bottom regions, thereby obtaining early vascular aggregates resting on solidified matrix.

4. The method of claim 3, wherein (viii) comprises after (vii.6)
(viii. 1) removing medium from the wells of CMCD(2) having early vascular aggregates resting on solidified matrix therein;
(viii.2) Embedding the early vascular aggregates resting on solidified matrix within the wells of CMCD(2) into matrix, said matrix comprising collagen, and solidifying the matrix having the early vascular aggregates embedded therein.

5. The method of any one of claims 1 to 4, wherein the first cell mass culturing device CMCD(1) comprises at least two wells, each having a culture space capable of storing the cell aggregate and culture fluid; each well having a tubular body which is disposed on the well on a plane at which the well has an opening and has an inner cavity communicating with the culture space; and
the first cell mass culturing device CMCD(1) further comprising a side wall which protrudes above openings of the wells and surrounds the wells, wherein the side wall is disposed on the outside of the tubular bodies, and each tubular body having one or more communication portion(s) formed within its tubular wall, said communication portion(s) capable of discharging a culture fluid and moving the culture fluid to an area between outside of the tubular bodies but within the side wall, wherein the communication portion(s) is/are slit(s) and/or through hole(s), which prevents passage of the cell aggregate to the outside of the tubular bodies.

6. The method of any one of claims 1 to 5, wherein for each well of the second cell mass culturing device CMCD(2), the lower section of said well has a smaller crosssectional area than the open upper section of the well, preferably a smaller cross-sectional area than the open upper end and/or the open lower end of the open upper section.

7. The method of any one of claims 1 to 6, wherein the aggregation medium of (i) comprises a knockout DMEM/F 12 medium, which preferably comprises one or more of KnockOut Serum replacement (KnockOut SR), L-glutamine, non-essential amino acids, β-mercapthoethanol and antibiotics;
and/or, preferably, and,
wherein the mesoderm induction medium of (iii) comprises a neurobasal medium, preferably supplemented with N2 and/or, preferably, and, B27 (N2B27 medium);
and/or, preferably, and,
wherein the medium suitable for inducing vascular differentiation (v) comprises a neurobasal medium, preferably supplemented with N2 and/or, preferably, and, B27 (N2B27 medium);
and/or, preferably and,
wherein the medium suitable to induce angiogenic sprouting of (ix) comprises a serum free medium and one or more of fetal bovine serum (FBS), human platelet lysate (hPL), Panexin CD (PCD), and mixtures of two or more thereof, wherein the angiogenesis medium preferably comprises at least PCD, more preferably PCD without FBS and/or hPL.

8. The method of any one of claims 1 to 7, wherein the matrix of (viii) comprises human collagen, preferably human collagen I; and/or, preferably and, wherein the matrix of (viii) comprises Matrigel; wherein the matrix of (viii) is preferably xeno reduced, more preferably is free of non-human collagen.

9. The method of any one of claims 1 to 8, wherein the matrix of (viii) further comprises hyaluronic acid as matrix forming component, wherein preferably, the mass based ratio of hyaluronic acid to matrigel is in the range of from 0.1 : 100 to 9 : 10, preferably in the range of from 10 : 90 to 90 : 10, more preferably in the range of from 1:1 to 7:3.

10. The method of any one of claims 1 to 9, wherein the induced pluripotent stem cells (iPSCs) are derived or derivable from mammalian somatic cells, preferably from mammalian somatic body tissue cells or mammalian somatic body fluid cells.

11. The method of any one of claims 1 to 10, which is an *ex vivo* method or *in vitro* method, preferably an *in vitro* method.

12. Three dimensional human blood vessel organoids obtained or obtainable from the method of any one of claims 1 to 11.

13. Three dimensional human blood vessel organoids of claim 12 for use in *(in vitro)* drug screening and/or *(in vitro)* drug testing.

14. Three dimensional human blood vessel organoids of claim 12 for use in *(in vitro)* toxicity analysis.

15. Three dimensional human blood vessel organoids of claim 12 for use in *(in vitro)* bioengineering; and/or for *(in vitro)* transplant preparation.
